# EUROPEAN PATENT APPLICATION

(11) **EP 4 706 839 A1**
(43) Date of publication of application: **11.03.2026**
(21) Application number: 25795382.8
(22) Date of filing: 28.08.2025
(51) Int. Cl.: B09B 3/00

(54) **CONTINUOUS PRODUCTION SYSTEM AND PROCESS FOR EFFICIENTLY RECOVERING PTA FROM WASTE PET, AND USE THEREOF**

(30) Priority: 13.03.2025 CN 202510293682
(71) Applicant: Yuantian Biotechnology (Tianjin) Co., Ltd., Tianjin 300457 (CN)
(72) Inventor: YOU, Shengping, Tianjin 300457 (CN); HAN, Dongdong, Tianjin 300457 (CN); LV, Wenjun, Tianjin 300457 (CN); GAO, Changbin, Tianjin 300457 (CN); LIU, Hailiang, Tianjin 300457 (CN); CHEN, Lei, Tianjin 300457 (CN); YANG, Jiyan, Tianjin 300457 (CN); XU, Zonghan, Tianjin 300457 (CN); JIANG, Gangwen, Tianjin 300457 (CN); WANG, Mengfan, Tianjin 300457 (CN)
(74) Representative: Isarpatent
(86) International application number: PCT/CN2025/117462
(87) International publication number: WO 2026/017180

(57) **Abstract**

The invention relates to the technical field of recovery from waste PET, and in particular to a continuous production system, a process and applications for efficient recovery of PTA from waste PET. Due to the cooperation of enzymatic hydrolysis cells, first solid-liquid separation unit, filter cake dissolution and decolorization unit, electrolysis cell, second solid-liquid separation unit, and recrystallization unit, the continuous production system and process provided in the invention can achieve the continuous production of PTA from waste PET. Furthermore, by adjusting the flow channel structure of the electrolysis assembly in the electrolysis cell, the deposition of TA which is prone to deposit on the anode plate caused by electrolysis can be avoided, thereby ensuring the continuous operation of the entire degradation and recovery process. This continuous production system and process are of great significance for the efficient degradation of waste PET and recovery.

## Description

### FIELD OF THE INVENTION

The invention relates to the technical field of recovery from waste PET, and in particular to a continuous production system, a process and applications for efficient recovery of PTA from waste PET.

### BACKGROUND OF THE INVENTION

Polyethylene terephthalate (PET) is widely used in various fields such as plastic bottles, food packaging, and textiles due to its excellent transparency, strength, and chemical resistance. However, the widespread use of PET materials has caused enormous pressure on the ecological environment. Therefore, achieving green recycling and effective utilization of PET is of great importance for promoting environmental protection and sustainable development.

In order to promote the recycling of resources, the discarded PET products are usually degraded to recover raw material monomer terephthalic acid (TA). At present, the recycling processes are mostly batch treatments. For example, the waste PET is degraded into terephthalic acid salt and ethylene glycol by chemical methods, and then the terephthalic acid salt is decolorized, performed acid adjustment and filtered to obtain high-purity purified terephthalic acid (PTA). However, these operation steps are complex and the production efficiency is low. There are also studies to obtain higher purity TA by electrolysis of terephthalic acid salt solution, but the process used is still batch processing, that is, it is necessary to use terephthalic acid salt solution as raw material for electrolysis, and it is not possible to directly use waste PET as the initial raw material of the whole process. Moreover, in the electrolysis process, the TA precipitated from the electrode reaction is easily deposited on the electrode surface, which affects the continuous progress of the electrolysis reaction, and may even lead to the interruption of the electrolysis reaction, which cannot ensure the continuity of the electrolysis process, resulting in low electrolysis efficiency.

Therefore, an urgent need exists for a process that can directly recover high-purity PTA from waste PET continuously, thereby achieving the continuous recycling of "waste PET to PTA".

### SUMMARY OF THE INVENTION

The invention provides a continuous production system, a process and applications for efficient recovery of PTA from waste PET directly. The continuous production system includes enzymatic hydrolysis cell, solid-liquid separation unit, electrolysis cell, recrystallization unit, and ethylene glycol recovery unit. Through the specific connection and coordination of each unit, as well as the special structure design of the electrolysis assembly in the electrolysis cell, the continuous production of PTA from waste PET is achieved, while ethylene glycol is also recovered.

A first aspect, the invention provides a continuous production system for efficient recovery of PTA from waste PET, and the continuous production system includes:
a plurality of enzymatic hydrolysis cells, configured to sequentially perform an enzymatic hydrolysis of PET to obtain a TA alkaline salt slurry;
a treatment cell, including a first solid-liquid separation unit, a filter cake dissolution and decolorization unit, an electrolysis cell, a second solid-liquid separation unit and a recrystallization unit which are connected in sequence; the treatment cell is configured to perform a solid-liquid separation of the TA alkaline salt slurry, a dissolution and decolorization, and an electrolysis to obtain a TA crude slurry which is subjected to a solid-liquid separation and recrystallization to obtain a PTA; and
a switching control unit, configured to connect the treatment cell with a first enzymatic hydrolysis cell of the plurality of enzymatic hydrolysis cells to initiate processing operation when the first enzymatic hydrolysis cell completes the enzymatic hydrolysis of PET, wherein during the processing operation of the first enzymatic hydrolysis cell by the treatment cell, a second enzymatic hydrolysis cell of the plurality of enzymatic hydrolysis cells performs the enzymatic hydrolysis of PET; in response to the treatment cell completing the processing operation on the first enzymatic hydrolysis cell, the switching control unit switches the treatment cell to connect with the second enzymatic hydrolysis cell, so that the treatment cell circularly connects with each enzymatic hydrolysis cell of the plurality of enzymatic hydrolysis cells to achieve continuous processing;
wherein the electrolysis cell comprises a plurality of electrolysis assemblies connected in parallel, and each electrolysis assembly is divided into an anode chamber and a cathode chamber by a cation exchange membrane. The anode chamber is equipped with an anode plate and two electrode plate frames arranged at both sides of the anode plate to form an anode cell, and the cathode chamber is equipped with a cathode plate and two electrode plate frames arranged at both sides of the cathode plate to form a cathode cell. A TA alkaline salt solution enters the electrolysis cell from the anode chamber, and the TA crude slurry is recovered in the anode cell, and an alkaline solution is recovered in the cathode cell. The anode cell and the cathode cell are hollow, in each of which two or more flow channels are provided along a fluid flow direction, and each flow channel is provided with a fluid inlet and a fluid outlet, where adjacent flow channels are separated by an arc-shaped corrugated separator perpendicular to the anode plate or the cathode plate, correspondingly.

The continuous production system for efficient recovery of PTA from waste PET provided in the invention includes a plurality of enzymatic hydrolysis cells, each of which is connected to the first solid-liquid separation unit, respectively, to continuously provide the first solid-liquid separation unit with the TA alkaline salt slurry obtained by the enzymatic hydrolysis of PET, thereby ensuring the continuous production of the system. After the enzymatic hydrolysis cell is connected to the first solid-liquid separation unit, the TA alkaline salt slurry enters the first solid-liquid separation unit for solid-liquid separation. The separated TA alkaline salt solid enters the filter cake dissolution and decolorization unit for dissolution, decolourization, removal of impurities and filtration. The filtered TA alkaline salt solution enters the electrolysis cell, and the TA crude slurry is recovered in the anode cell of the electrolysis cell, and an alkaline solution is recovered in the cathode cell of the electrolysis cell. The obtained TA crude slurry enters the second solid-liquid separation unit to filter to collect a solid TA crude product, which is refined by the recrystallization unit to obtain the PTA.

Combining the first aspect, the first solid-liquid separation unit is configured to separate the TA alkaline salt slurry to obtain a TA alkaline salt; the filter cake dissolution and decolorization unit is configured to dissolve, decolorize and filter the TA alkaline salt to obtain the TA alkaline salt solution; the second solid-liquid separation unit is configured to separate the TA crude slurry to obtain a TA crude product; and the recrystallization unit is configured to refine the TA crude product.

Combining the first aspect, the continuous production system further includes a distillation unit connected to a solution side of the first solid-liquid separation unit for recovering ethylene glycol in the solution.

A second aspect, the invention provides a continuous production process for efficient recovery of PTA from waste PET, and the continuous production process includes the following steps:
an enzymatic hydrolysis step, comprising a plurality of enzymatic hydrolysis cells; performing the enzymatic hydrolysis using a PET-degrading enzyme, an enzymatic hydrolysis temperature being 40°C to 70°C, and an enzymatic hydrolysis pH being 8 to 10, to obtain a TA alkaline salt slurry;
a first solid-liquid separation step, comprising separating the TA alkaline salt slurry to obtain a TA alkaline salt;
a filter cake dissolution and decolorization step, comprising adding water with a weight of 5 to 10 times of the TA alkaline salt to the TA alkaline salt to dissolve the TA alkaline salt, adding a decolorant, and filtering to obtain a TA alkaline salt solution;
an electrolysis step, including a plurality of electrolysis assemblies connected in parallel, and each electrolysis assembly is divided into an anode chamber and a cathode chamber by a cation exchange membrane, wherein the anode chamber is equipped with an anode plate and two electrode plate frames arranged at both sides of the anode plate to form an anode cell, and the cathode chamber is equipped with a cathode plate and two electrode plate frames arranged at both sides of the cathode plate to form a cathode cell; the TA alkaline salt solution enters the electrolysis assembly from an anode side, and a TA crude slurry is recovered in the anode cell, and an alkaline solution is recovered in the cathode cell, and oxygen and hydrogen are collected separately; each electrode plate frame is in a rectangular shape, wherein one or more arc-shaped corrugated separator is provided along a long side direction of the electrode plate frame, and a curvature diameter of the arc-shaped corrugated separator is not less than a short side size of the cell (anode cell or cathode cell, the same below);
a second solid-liquid separation step, comprising filtering the TA crude slurry to obtain a TA crude product; and
a refining step, comprising recrystallizing the TA crude product to obtain the PTA;
when a first enzymatic hydrolysis cell of the plurality of enzymatic hydrolysis cells completes the enzymatic hydrolysis step and performs the first solid-liquid separation step, a second enzymatic hydrolysis cell of the plurality of enzymatic hydrolysis cells performs the enzymatic hydrolysis step; and after the processing operation on the first enzymatic hydrolysis cell is completed, the second enzymatic hydrolysis cell performs the first solid-liquid separation step, thereby achieving continuous production.

The continuous production process for efficient recovery PTA from waste PET provided in this invention achieves continuous production of PTA using waste PET as raw material through an enzymatic hydrolysis step, a first solid-liquid separation step, a filter cake dissolution and decolorization step, an electrolysis step, a second solid-liquid separation step, and a refining step. Specifically, an enzymatic hydrolysis is performed on the pretreated waste PET with a PET-degrading enzyme to obtain the TA alkaline salt slurry. Due to the low solubility of the TA alkaline salt in the crude enzyme solution used for enzymatic hydrolysis, the TA alkaline salt will gradually precipitate as the degree of enzymatic hydrolysis increases. The TA alkaline salt slurry is separated in the first solid-liquid separation step to obtain the solid TA alkaline salt. The TA alkaline salt filter cake is dissolved in a dissolvable amount of water, a decolorant is added for decolorization and removal of impurities, and the decolorant is removed at last. The obtained TA alkaline salt solution is electrolyzed to obtain the TA crude slurry at the anode of the electrolysis assembly and obtain the alkaline solution at the cathode of the electrolysis assembly. The TA crude slurry generated at the anode of each electrolysis assembly is separated in the second solid-liquid separation step to collect the solid to obtain the TA crude product, which is further recrystallized to obtain the PTA. When a first enzymatic hydrolysis cell of the plurality of enzymatic hydrolysis cells completes the enzymatic hydrolysis step and performs the first solid-liquid separation step, a second enzymatic hydrolysis cell of the plurality of enzymatic hydrolysis cells performs the enzymatic hydrolysis step; and after the processing operation on the first enzymatic hydrolysis cell is completed, the second enzymatic hydrolysis cell performs the first solid-liquid separation step, thereby achieving continuous production.

Combining the second aspect, the continuous production process further includes a distillation step, comprising distilling a solution obtained from the first solid-liquid separation step to recover ethylene glycol.

Combining the second aspect, the enzymatic hydrolysis step includes: preparing 10 mmol/L to 1000 mmol/L sodium phosphate buffer, adding 100g to 1000g waste PET per liter of the buffer, and adding PET-degrading enzyme crude enzyme solution with a mass of 0.1% to 1% of the buffer, to perform the enzymatic hydrolysis for 5h to 25h.

The refining step includes: transporting the TA crude product to a slurry tank, and adding a mixed solvent to adjust a mass concentration of the TA crude product to 20wt% to 35wt%, wherein the mixed solvent is a mixture of water and at least one of dimethylformamide (DMF), diethylformamide (DEF) and dimethyl sulfoxide (DMSO); heating to 265°C to 280°C and pressurizing to 6.0 MPa to 8.0 MPa to dissolve the TA crude product; performing decolorization and removal of metal impurities on an obtained TA crude solution through an adsorption column, subjecting to stepwise crystallization through a 1 to 6 stage cooling-depressurization flash crystallizer, washing and drying to obtain the PTA.

For example, at least one of DMF, DEF and DMSO is mixed with desalted water to form the mixed solvent, where a mass ratio of the desalted water to the mixed solvent is 80% to 95%.

Combining the second aspect, a ratio of a long side size to the short side size of the cell is 2:1 to 5:1, and a depth of the cell is 2 mm to 20 mm; a distance between an electrode plate upper edge and an upper edge of the cell is greater than 1/8 of the long side size of the cell, and a distance between an electrode plate lower edge and a lower edge of the cell is not less than 1/4 of the long side size of the cell.

A PTA is produced using the continuous production system for efficient recovery of PTA from waste PET or according to the continuous production process for efficient recovery of PTA from waste PET as described above.

An application of the PTA in the preparation of PET products, and the PET products include PET films, PET fibers, and/or bottle grade PET chips.

For example, the PET films prepared by the PTA can be applied in a plurality of fields and industries, such as food packaging films, heat sealable films, and stretchable films in the packaging industry; such as electronic protective films, light-diffusing films, and anti-reflection films in the electronic and optical fields; as well as industrial and functional films, such as anti-static films, high-temperature resistant films, and electrical insulation films. They can also be used for biaxially stretchable films or unidirectionally stretchable films.

For example, the PET fibers prepared by the PTA can also be applied in a plurality of fields and industries, such as polyester filament fibers or polyester staple fibers produced in the textile industry; such as high-strength fibers, low-elasticity fibers or high-modulus fibers commonly used in the industrial field. Additionally, they can be used for functional fibers such as moisture wicking fibers, anti-static fibers, UV-resistant fibers or flame-retardant fibers. They can also be pre-oriented yarns, fully drawn yarns or tensile deformation yarns produced according to different production processes.

For example, the bottle grade PET chips prepared by the PTA can also be applied in a plurality of fields and industries, such as food packaging bottles, beverage packaging bottles, medicine packaging bottles, cosmetic packaging bottles, or industrial chemical bottles. The bottle grade PET chips can be with different structures, such as single-layer bottle grade PET chips, multi-layer bottle grade PET chips, or composite bottle grade PET chips. The bottle grade PET chips can be with specific functions, such as UV-resistant bottle grade PET chips, high-temperature resistant bottle grade PET chips, low-temperature resistant bottle grade PET chips, or high-barrier bottle grade PET chips.

The continuous production system and process provided in this invention can achieve the continuous production of PTA from waste PET. Furthermore, by adjusting the flow channel structure of the electrolysis assembly, the deposition of TA which is prone to deposit on the anode plate caused by electrolysis can be avoided, thereby ensuring the continuous operation of the entire degradation and recovery process. This continuous production system and process are of great significance for the efficient degradation of waste PET and recovery.

### BRIEF DESCRIPTION OF THE DRAWINGS

Fig. 1 shows a continuous production process for efficient recovery of PTA from waste PET according to the invention;
Fig. 2 shows a schematic diagram of an electrolysis cell and a second solid-liquid separation unit of a continuous production process for efficient recovery of PTA from waste PET according to the invention;
Fig. 3 shows a structure of an electrolysis assembly in an electrolysis cell of a continuous production process for efficient recovery of PTA from waste PET according to the invention;
Fig. 4(a) is a cross-sectional view of an anode chamber in an electrolysis assembly of an electrolysis cell, and Fig. 4(b) is a cross-sectional view of a cathode chamber in the electrolysis assembly of the electrolysis cell; and
Fig. 5 shows a refining process of a continuous production process for efficient recovery of PTA from waste PET according to the invention;

In the drawings:
021, cathode chamber; 022, anode chamber; 023, cation exchange membrane; 024, cathode plate; 025, anode plate; 026, electrode plate frame; 027, sealing gasket; 028, end plate;
301, electrode plate upper edge; 302, electrode plate lower edge;
400, arc-shaped corrugated separator; 401, first flow channel; 402, second flow channel;
601, anode chamber fluid inlet; 602, anode chamber fluid outlet; 701, anode plate frame upper edge fluid pipe; 702, anode plate frame lower edge fluid pipe; 801, cathode chamber fluid inlet; 802, cathode chamber fluid outlet; 901, cathode plate frame upper edge fluid pipe; 902, cathode plate frame lower edge fluid pipe.

### DETAILED DESCRIPTION OF THE INVENTION

In order to make the technical problem, technical solution and advantages of the invention more clearly understood, further details are described in conjunction with the drawings and examples in the invention. It should be understood that the specific examples described herein are only used to interpret the invention and are not intended to limit the invention.

In this invention, unless otherwise specified, the use of terms such as "first", "second", or "third" is intended to distinguish different objects and not to describe a specific order.

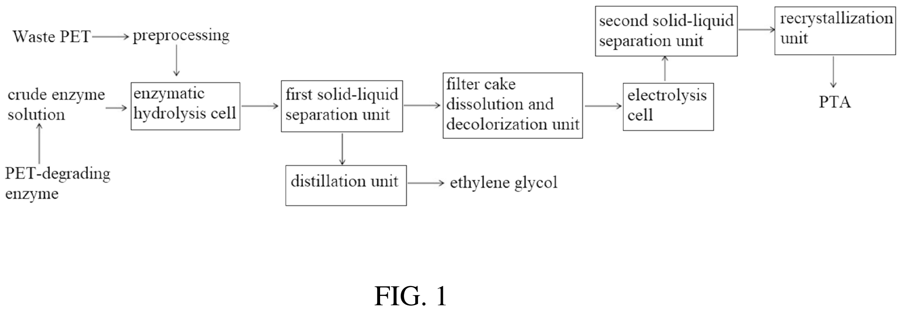

Referring to Fig. 1, a continuous production system for efficient recovery of PTA from waste PET provided by the invention will be described. The continuous production system includes: a plurality of enzymatic hydrolysis cells, configured to sequentially perform an enzymatic hydrolysis of PET to obtain a TA alkaline salt slurry; a treatment cell, including a first solid-liquid separation unit, a filter cake dissolution and decolorization unit, an electrolysis cell, a second solid-liquid separation unit and a recrystallization unit which are connected in sequence; the treatment cell is configured to perform a solid-liquid separation of the TA alkaline salt slurry, a dissolution and decolorization, and an electrolysis to obtain a TA crude slurry which is subjected to a solid-liquid separation and recrystallization to obtain a PTA; and a switching control unit, configured to connect the treatment cell with a first enzymatic hydrolysis cell of the plurality of enzymatic hydrolysis cells to initiate processing operation when the first enzymatic hydrolysis cell completes the enzymatic hydrolysis of PET, wherein during the processing operation of the first enzymatic hydrolysis cell by the treatment cell, a second enzymatic hydrolysis cell of the plurality of enzymatic hydrolysis cells performs the enzymatic hydrolysis of PET; in response to the treatment cell completing the processing operation on the first enzymatic hydrolysis cell, the switching control unit switches the treatment cell to connect with the second enzymatic hydrolysis cell, so that the treatment cell circularly connects with each enzymatic hydrolysis cell of the plurality of enzymatic hydrolysis cells to achieve continuous processing.

The continuous production system for efficient recovery of PTA from waste PET provided in the invention includes a plurality of enzymatic hydrolysis cells, each of which is connected to the first solid-liquid separation unit, respectively, to continuously provide the first solid-liquid separation unit with the TA alkaline salt slurry obtained by the enzymatic hydrolysis of PET, thereby ensuring the continuous production of the system. Each enzymatic hydrolysis cell has the same subsequent processing operations. The subsequent processing operations after connecting one of the plurality of enzymatic hydrolysis cells (e.g., a first enzymatic hydrolysis cell) to the treatment cell are described. After the first enzymatic hydrolysis cell is connected to the first solid-liquid separation unit, the TA alkaline salt slurry enters the first solid-liquid separation unit for solid-liquid separation. The separated TA alkaline salt solid enters the filter cake dissolution and decolorization unit for dissolution, decolourization, removal of impurities and filtration. The filtered TA alkaline salt solution enters the electrolysis cell, and the TA crude slurry is recovered in the anode cell of the electrolysis cell, and an alkaline solution is recovered in the cathode cell of the electrolysis cell. The obtained TA crude slurry enters the second solid-liquid separation unit to filter to collect a solid TA crude product, which is refined by the recrystallization unit to obtain the PTA.

As a specific embodiment of a continuous production system for efficient recovery of PTA from waste PET in this invention, the continuous production system further includes a control unit, which is connected to the liquid outlet ends of the plurality of enzymatic hydrolysis cells. After the enzymatic hydrolysis solution of one of the plurality of enzymatic hydrolysis cells is processed by the treatment cell, the control unit controls the liquid outlet end of another enzymatic hydrolysis cell to be connected to the first solid-liquid separation unit.

The control unit is provided to control the connection between the plurality of enzymatic hydrolysis cells and the first solid-liquid separation unit, so as to ensure that the enzymatic hydrolysis solution generated by the plurality of enzymatic hydrolysis cells is sufficient for supplying the subsequent processing of each unit, and ensure continuous production of the whole continuous production system.

As a specific embodiment of a continuous production system for efficient recovery of PTA from waste PET in this invention, the continuous production system further includes a distillation unit connected to a solution side of the first solid-liquid separation unit for recovering ethylene glycol in the solution.

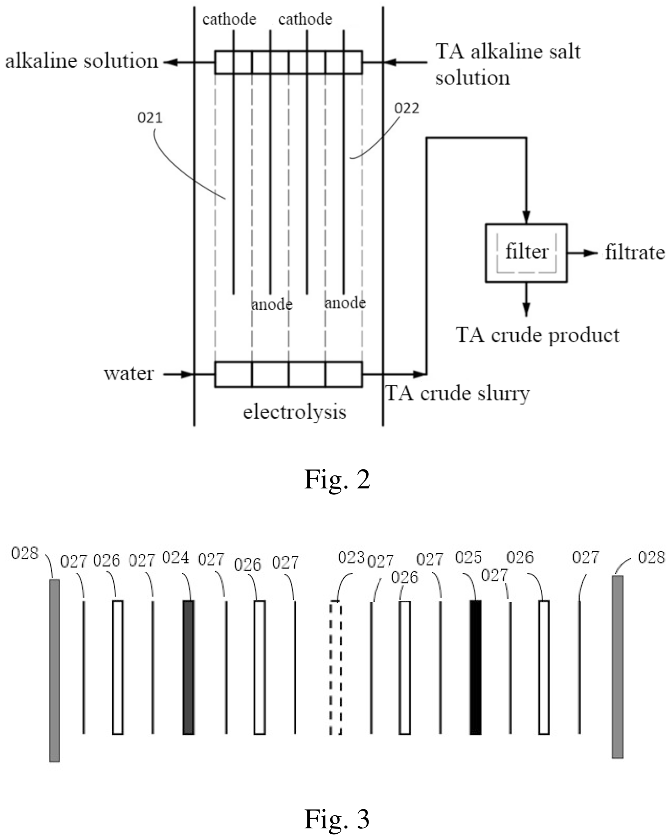

In Fig. 2, as a specific embodiment of a continuous production system for efficient recovery of PTA from waste PET in this invention, the electrolysis cell includes at least two electrolysis assemblies, consisting of an alternating arrangement of anodes and cathodes in the electrolysis assemblies. The TA alkaline salt solution (i.e., decolorization solution) generated in the filter cake dissolution and decolorization unit enters the electrolysis cell from the anode side, and the alkaline solution generated by electrolysis flows out from the cathode side.

In Fig. 3, as a specific embodiment of a continuous production system for efficient recovery of PTA from waste PET in this invention, a sealing gasket 027 is installed between the cation exchange membrane 023 and the electrode plate frame 026, between the electrode plate frame 026 and the cathode plate 024 or between the electrode plate frame 026 and the anode plate 025, between the electrode plate frame 026 and the end plate 028 in each electrolysis assembly. Each module is fixed by screws that pass through and nuts at both ends of the screws.

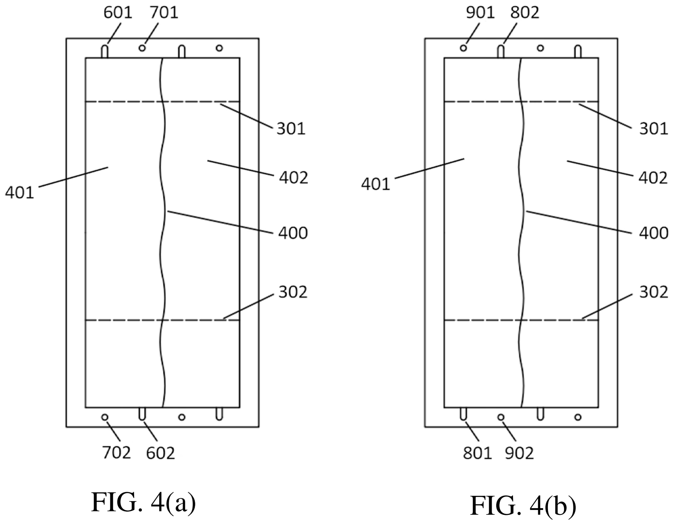

As a specific embodiment of a continuous production system for efficient recovery of PTA from waste PET in this invention, the electrode plate frames 026 are set on both sides of the anode plate 025 and both sides of the cathode plate 024, respectively, forming the anode cell and the cathode cell, that is the electrode plate frames 026 parallel to the anode plate 025 form the anode cell, and the electrode plate frames 026 parallel to the cathode plate 024 form the cathode cell. The anode cell and the cathode cell are hollow, in each of which two or more flow channels are provided along a fluid flow direction, and each flow channel is provided with a fluid inlet and a fluid outlet, where adjacent flow channels are separated by an arc-shaped corrugated separator 400 perpendicular to the anode plate 025 or the cathode plate 024, correspondingly. The specific structure is as shown in Figs. 4(a) and 4(b).

In Fig. 4(a), as a specific embodiment of a continuous production system for efficient recovery of PTA from waste PET in this invention, in the hollow anode cell formed by the anode plate 025 and the electrode plate frame 026, at least two flow channels (a first flow channel 401 and a second flow channel 402) are provided along the direction of fluid flow. The adjacent flow channels are separated by an arc-shaped corrugated separator 400, which is perpendicular to the anode plate 025. Each flow channel is equipped with an anode chamber fluid inlet 601 and an anode chamber fluid outlet 602, and an anode plate frame upper edge fluid pipe 701 and an anode plate frame lower edge fluid pipe 702 are respectively set at the upper edge of the electrode plate frame and the lower edge of the electrode plate frame corresponding to each flow channel. The anode plate 025 is installed between the electrode plate upper edge 301 and the electrode plate lower edge 302. In Fig. 4(b), in the hollow cathode cell formed by the cathode plate 024 and the electrode plate frame 026, at least two flow channels (a first flow channel 401 and a second flow channel 402) are provided along the direction of fluid flow. The adjacent flow channels are separated by an arc-shaped corrugated separator 400, which is perpendicular to the cathode plate 024. Each flow channel is equipped with a cathode chamber fluid inlet 801 and a cathode chamber fluid outlet 802, and a cathode plate frame upper edge fluid pipe 901 and a cathode plate frame lower edge fluid pipe 902 are respectively set at the upper edge of the electrode plate frame and the lower edge of the electrode plate frame corresponding to each flow channel. The cathode plate 024 is installed between the electrode plate upper edge 301 and the electrode plate lower edge 302.

The arc-shaped corrugated separator 400 is used to separate the adjacent flow channels. When the liquid flows in the anode cell, it collides with the flow channel with a certain curvature separated by the arc-shaped corrugated separator 400, increasing its own flow velocity. The increased flow velocity of the liquid has a greater impact force on the TA crude slurry generated on the surface of the anode plate 025, making it unable to deposit stably on the surface of the anode plate 025.

When electrolysis is carried out using an electrolysis cell including two electrolysis assembly in parallel connection, the TA alkaline salt solution (i.e., decolorization solution) enters the anode chamber 022 through the anode chamber fluid inlet 601, or the TA alkaline salt solution flows through the cathode plate frame upper edge fluid pipe 901 and then enters the anode chamber 022 through the anode chamber fluid inlet 601. The TA alkaline salt combines with the hydrogen ions generated by electrolysis in anode in the anode chamber 022 to form a TA crude product. The TA crude slurry flows out from the anode chamber fluid outlet 602 and is mixed with the TA crude slurry flowing out from the anode chamber 022 of another electrolysis assembly through the cathode plate frame lower edge fluid pipe 902 before entering the main pipe, and oxygen escapes and is collected. At the same time, the water required for electrolysis enters the cathode chamber 021 through the cathode chamber fluid inlet 801 and the anode plate frame lower edge fluid pipe 702. Hydroxide ions generated by electrolysis water in cathode and sodium ions or potassium ions entering the cathode chamber 021 through the cation exchange membrane 023 generate an alkaline solution, which flows out from the cathode chamber fluid outlet 802 and is mixed with the alkaline solution flowing out from the cathode chamber 021 of another electrolysis assembly through the anode plate frame upper edge fluid pipe 701 before entering the main pipe.

The second aspect, the invention provides a continuous production process for efficient recovery of PTA from waste PET, and the continuous production process includes the following steps:
an enzymatic hydrolysis step, comprising a plurality of enzymatic hydrolysis cells; performing the enzymatic hydrolysis using a PET-degrading enzyme to hydrolyze waste PET, an enzymatic hydrolysis temperature being 40°C to 70°C, and an enzymatic hydrolysis pH being 8 to 10, to obtain a TA alkaline salt slurry;
a first solid-liquid separation step, comprising separating the TA alkaline salt slurry to obtain a TA alkaline salt;
a filter cake dissolution and decolorization step, comprising adding water with a weight of 5 to 10 times of the TA alkaline salt to the TA alkaline salt to dissolve the TA alkaline salt, adding a decolorant, and filtering to obtain a TA alkaline salt solution;
an electrolysis step, including a plurality of electrolysis assemblies connected in parallel, and each electrolysis assembly is divided into an anode chamber and a cathode chamber by a cation exchange membrane. The TA alkaline salt solution enters the electrolysis assembly from an anode side, and a TA crude slurry is recovered in the anode, and an alkaline solution is recovered in the cathode, and oxygen and hydrogen are collected separately;
a second solid-liquid separation step, comprising filtering the TA crude slurry to obtain a TA crude product; and
a refining step, comprising recrystallizing the TA crude product to obtain the PTA.

The continuous production process for efficient recovery PTA from waste PET provided in this invention achieves continuous production of PTA using waste PET as raw material through an enzymatic hydrolysis step, a first solid-liquid separation step, a filter cake dissolution and decolorization step, an electrolysis step, a second solid-liquid separation step, and a refining step. Specifically, an enzymatic hydrolysis is performed on the pretreated waste PET with a PET-degrading enzyme to obtain the TA alkaline salt slurry. Due to the low solubility of the TA alkaline salt in the crude enzyme solution used for enzymatic hydrolysis, the TA alkaline salt will gradually precipitate as the degree of enzymatic hydrolysis increases. The TA alkaline salt slurry is separated in the first solid-liquid separation step to obtain the solid TA alkaline salt. The TA alkaline salt filter cake is dissolved in a dissolvable amount of water, a decolorant is added for decolorization and removal of impurities, and the decolorant is removed at last. The obtained TA alkaline salt solution is electrolyzed to obtain the TA crude slurry at the anode of the electrolysis assembly and obtain the alkaline solution at the cathode of the electrolysis assembly. The TA crude slurry generated at the anode of each electrolysis assembly is separated in the second solid-liquid separation step to collect the solid to obtain the TA crude product, which is further recrystallized to obtain the PTA.

As a specific embodiment of a continuous production process for efficient recovery of PTA from waste PET in this invention, the continuous production process further includes a TA alkaline salt slurry control step, comprising after the TA alkaline salt slurry in one of the plurality of enzymatic hydrolysis cells is processed, the TA alkaline salt slurry in the other of the plurality of enzymatic hydrolysis cells is controlled to undergo the first solid-liquid separation step.

As a specific embodiment of a continuous production process for efficient recovery of PTA from waste PET in this invention, the continuous production process further includes a distillation step, comprising distilling a solution obtained from the first solid-liquid separation step to recover ethylene glycol.

As a specific embodiment of a continuous production process for efficient recovery of PTA from waste PET in this invention, the enzymatic hydrolysis step includes: preparing 10 mmol/L to 1000 mmol/L sodium phosphate buffer, adding 100g to 1000g waste PET per kilogram of the buffer, and adding PET-degrading enzyme crude enzyme solution with a mass of 0.1% to 1% of the buffer, to perform the enzymatic hydrolysis for 5h to 25h.

After the enzymatic hydrolysis step is completed, in order to precipitate more TA alkaline salts, the slurry can be cooled appropriately according to the actual temperature before the first solid-liquid separation step.

As a specific embodiment of a continuous production process for efficient recovery of PTA from waste PET in this invention, each electrolysis assembly is divided into an anode chamber 022 and a cathode chamber 021 by a cation exchange membrane 023, wherein the anode chamber 022 is equipped with an anode plate 025 and two electrode plate frames 026 arranged at both sides of the anode plate 025 to form an anode cell, and the cathode chamber 021 is equipped with a cathode plate 024 and two electrode plate frames 026 arranged at both sides of the cathode plate 024 to form a cathode cell. The TA crude slurry is recovered in the anode cell, and an alkaline solution is recovered in the cathode cell. Since the two electrode plate frames 026 in the anode chamber 022 and the two electrode plate frames 026 in the cathode chamber 021 are completely the same, the anode cell and the cathode cell have the same size. The term "cell" mentioned in the following description refers to the anode cell and/or the cathode cell. The electrode plate frame is of a rectangular shape. The long side size (length) of the cell is the long side size of the electrode plate frame minus the upper and lower edges sizes of the electrode plate frame in the long side direction, the short side size (width) of the cell is the short side size of the electrode plate frame minus the left and right edges sizes of the electrode plate frame in the short side direction, and the depth of the cell is the horizontal width of two parallel electrode plate frames clamping the electrode plate. One or more arc-shaped corrugated separator 400 is provided along a long side direction of the electrode plate frame, and a curvature diameter of the arc-shaped corrugated separator 400 is not less than a short side size of the cell. A ratio of a long side size to the short side size of the cell is 2:1 to 5:1, and a depth of the cell is 2 mm to 20 mm; a distance between an electrode plate upper edge 301 and an upper edge of the cell is greater than 1/8 of the long side size of the cell, and a distance between an electrode plate lower edge 302 and a lower edge of the cell is not less than 1/4 of the long side size of the cell.

Specifically, the length of the electrode plate frame is 30 cm, and the width is 10 cm. The length of the cell is 26 cm, the width is 6 cm, and the depth is 5 mm. The curvature diameter of the arc-shaped corrugated separator 400 is 6 cm. The distance between the electrode plate upper edge 301 and the upper edge of the cell is 3.5 cm, and the distance between the electrode plate lower edge 302 and the lower edge of the cell is 6.5 cm.

As a specific embodiment of a continuous production process for efficient recovery of PTA from waste PET in this invention, the cathode plate 024 or the anode plate 025 is at least one of titanium plated ruthenium, titanium plated iridium, or titanium plated platinum. The voltage between the cathode plate 024 and the anode plate 025 is 1.5 Volts(V) to 5V.

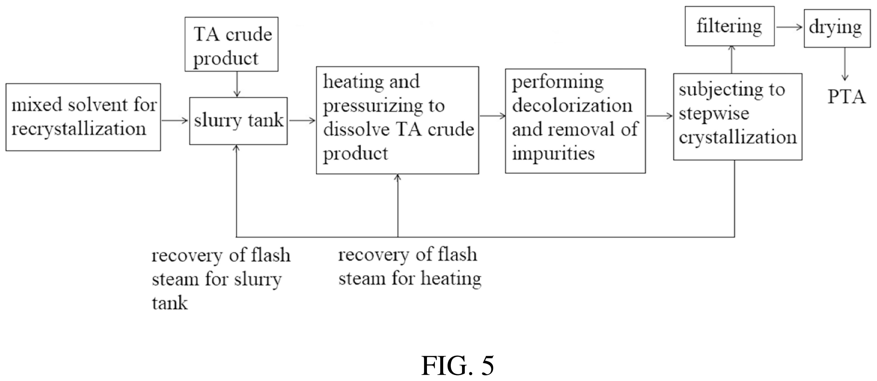

In Fig. 5, as a specific embodiment of a continuous production process for efficient recovery of PTA from waste PET in this invention, the refining step includes: transporting the TA crude product to a slurry tank, and adjusting a mass concentration of the TA crude product to 20wt% to 35wt% using a mixture of desalted water and at least one of DMF, DEF and DMSO; heating to 265°C to 280°C and pressurizing to 6.0 MPa to 8.0 MPa to dissolve the TA crude product; performing decolorization and removal of metal impurities on an obtained TA crude solution through an adsorption column, subjecting to stepwise crystallization through a 1 to 6 stage cooling-depressurization flash crystallizer; filtering the crystallized slurry through a filtration system, washing the filtered solid and drying to obtain the PTA.

As a specific embodiment of a continuous production process for efficient recovery of PTA from waste PET in this invention, the specific parameters for the stepwise crystallization process are: the temperature of the first stage crystallizer ranges from 250°C to 260°C, and the pressure ranges from 4.0 MPa to 4.5 MPa; the temperature of the second stage crystallizer ranges from 200°C to 210°C, and the pressure ranges from 3.0 MPa to 4.0 MPa; the temperature of the third stage crystallizer ranges from 160°C to 185°C, and the pressure ranges from 0.7 MPa to 1.1 MPa; the temperature of the fourth stage crystallizer ranges from 130°C to 155°C, and the pressure ranges from 0.3 MPa to 0.65 MPa.

As a specific embodiment of a continuous production process for efficient recovery of PTA from waste PET in this invention, the performing decolorization and removal of metal impurities on an obtained TA crude solution through an adsorption column includes: a combination of one or more processes such as fixed bed continuous adsorption, precision filtration, and membrane separation can be used to remove colored substances and metal impurities from the TA crude solution. The adsorbent material can be at least one of coconut charcoal, zeolite molecular sieve, diatomaceous earth, or chitosan, or other adsorbent materials with color removal and metal impurity adsorption functions can be selected.

In Fig. 5, as a specific embodiment of a continuous production process for efficient recovery of PTA from waste PET in this invention, in the refining step, the flash steam generated by the cooling-depressurization flash crystallizer can be passed into the front-end process of dissolving TA crude product and slurry tank, achieving energy recovery and utilization.

The continuous production process and system for efficient recovery PTA from waste PET provided in this invention are explained through specific examples as follows:
1000 mmol/L sodium phosphate buffer with a pH of 9.0 was prepared. 1000g pre-crushed waste PET was added to per kilogram of the buffer, and PET-degrading enzyme crude enzyme solution with a mass of 1wt% of the buffer was added to perform the enzymatic hydrolysis at 40°C to 70°C for 5h to 25h. The sodium hydroxide solution with a mass fraction of 5% to 50% was added dropwise to maintain the pH of the system ranging from 8 to 10 during the enzymatic hydrolysis process.

After the enzymatic hydrolysis was completed, the enzymatic hydrolysis solution was naturally cooled to room temperature and filtered. The filtrate entered a distillation unit for recovery of ethylene glycol, and the filter cake of TA alkaline salt weighted 1531g. 11 kg of water was added to the TA alkaline salt to dissolve it, and then 30g of decolorant was added and stirred for 1h for decolorization and removal of impurities. The decolorant and other insoluble were removed by filtration, and the filtrate entered the electrolysis cell. During the electrolysis process, the electrode voltage was set at 2V, and the current was set at 77.0A which was kept constant. After 9h of electrolysis reaction, the electrode voltage increased to 2.2V, which indicated that the deposited amount of the produced TA crude slurry on the surface of the anode plate 025 was minimal, and would not affect the electrolysis reaction. The TA crude slurry was filtered to obtain solid TA crude product which was further recrystallized. The recrystallization process included: 1200g of TA crude product was transported to a slurry tank through a spiral pipe, and 2369g of a mixed solvent was added to prepare a TA slurry (the mixed solvent was prepared by mixing desalted water and DMF in a mass ratio of 85:15). The temperature was heated to 280°C, and the pressure increased to 6.4 MPa to dissolve the TA crude product. The decolorization and removal of metal impurities was performed on the TA crude solution via an adsorption column filled with coconut charcoal, and the treated TA crude solution was subjected to stepwise crystallization through a 1 to 4 stage cooling-depressurization flash crystallizer. The specific parameters for the stepwise crystallization process were: the temperature of the first stage crystallizer ranged from 250°C to 260°C, and the pressure ranged from 4.0 MPa to 4.5 MPa; the temperature of the second stage crystallizer ranged from 200°C to 210°C, and the pressure ranged from 3.0 MPa to 4.0 MPa; the temperature of the third stage crystallizer ranged from 160°C to 185°C, and the pressure ranged from 0.7 MPa to 1.1 MPa; the temperature of the fourth stage crystallizer ranged from 130°C to 155°C, and the pressure ranged from 0.3 MPa to 0.65 MPa. The crystallized slurry was filtered through a filtration system, washed and dried to obtain 811g of PTA with a purity of 99.91% and a yield of 93.8%, wherein the yield (%) was calculated by the formula: 811/(1000/192 ×166)=93.8%.

However, when the mixed solvent used for recrystallization dissolution in the refining step was replaced with a mixed solvent of water, ethanol, isopropanol in a mass ratio of 80:10:10, the yield of PTA obtained decreased to 91.5% and the purity decreased to 98.4%, indicating that the above-mentioned recrystallization solvent used in this invention can ensure a high purity and yield of PTA.

The foregoing descriptions are only the preferred examples of the invention, but are not intended to limit the invention. Any modifications, equivalent substitutions, or improvements made within the spirit and principles of the invention shall fall within the scope of protection of the invention.

## Claims

1. A continuous production system for efficient recovery of purified terephthalic acid, PTA, from waste polyethylene terephthalate, PET, comprising:
a plurality of enzymatic hydrolysis cells, configured to sequentially perform an enzymatic hydrolysis of PET to obtain a terephthalic acid, TA, alkaline salt slurry;
a treatment cell, comprising a first solid-liquid separation unit, a filter cake dissolution and decolorization unit, an electrolysis cell, a second solid-liquid separation unit and a recrystallization unit which are connected in sequence; the treatment cell is configured to perform a solid-liquid separation of the TA alkaline salt slurry, a dissolution and decolorization, and an electrolysis to obtain a TA crude slurry which is subjected to a solid-liquid separation and recrystallization to obtain the PTA; and
a switching control unit, configured to connect the treatment cell with a first enzymatic hydrolysis cell of the plurality of enzymatic hydrolysis cells to initiate processing operation when the first enzymatic hydrolysis cell completes the enzymatic hydrolysis of PET, wherein during the processing operation of the first enzymatic hydrolysis cell by the treatment cell, a second enzymatic hydrolysis cell of the plurality of enzymatic hydrolysis cells performs the enzymatic hydrolysis of PET; in response to the treatment cell completing the processing operation on the first enzymatic hydrolysis cell, the switching control unit switches the treatment cell to connect with the second enzymatic hydrolysis cell, so that the treatment cell circularly connects with each enzymatic hydrolysis cell of the plurality of enzymatic hydrolysis cells to achieve continuous processing;
wherein the electrolysis cell comprises a plurality of electrolysis assemblies connected in parallel, and each electrolysis assembly is divided into an anode chamber and a cathode chamber by a cation exchange membrane, wherein the anode chamber is equipped with an anode plate and two electrode plate frames arranged at both sides of the anode plate to form an anode cell, and the cathode chamber is equipped with a cathode plate and two electrode plate frames arranged at both sides of the cathode plate to form a cathode cell; a TA alkaline salt solution enters the electrolysis cell from the anode chamber, and the TA crude slurry is recovered in the anode cell, and an alkaline solution is recovered in the cathode cell; the anode cell and the cathode cell are hollow, in each of which two or more flow channels are provided along a fluid flow direction, and each flow channel is provided with a fluid inlet and a fluid outlet, wherein adjacent flow channels are separated by an arc-shaped corrugated separator perpendicular to the anode plate or the cathode plate, correspondingly.

2. The continuous production system for efficient recovery of the PTA from the waste PET of claim 1, wherein the first solid-liquid separation unit is configured to separate the TA alkaline salt slurry to obtain a TA alkaline salt;
the filter cake dissolution and decolorization unit is configured to dissolve, decolorize and filter the TA alkaline salt to obtain the TA alkaline salt solution;
the second solid-liquid separation unit is configured to separate the TA crude slurry to obtain a TA crude product; and
the recrystallization unit is configured to refine the TA crude product.

3. The continuous production system for efficient recovery of the PTA from the waste PET of claim 1, further comprising a distillation unit connected to a solution side of the first solid-liquid separation unit for recovering ethylene glycol in the solution.

4. A continuous production process for efficient recovery of purified terephthalic acid, PTA, from waste polyethylene terephthalate, PET, comprising:
an enzymatic hydrolysis step, comprising a plurality of enzymatic hydrolysis cells; performing the enzymatic hydrolysis using a PET-degrading enzyme, an enzymatic hydrolysis temperature being 40°C to 70°C, and an enzymatic hydrolysis pH being 8 to 10, to obtain a terephthalic acid, TA, alkaline salt slurry;
a first solid-liquid separation step, comprising separating the TA alkaline salt slurry to obtain a TA alkaline salt;
a filter cake dissolution and decolorization step, comprising adding water with a weight of 5 to 10 times of the TA alkaline salt to the TA alkaline salt to dissolve the TA alkaline salt, adding a decolorant, and filtering to obtain a TA alkaline salt solution;
an electrolysis step, comprising a plurality of electrolysis assemblies connected in parallel, and each electrolysis assembly is divided into an anode chamber and a cathode chamber by a cation exchange membrane, wherein the anode chamber is equipped with an anode plate and two electrode plate frames arranged at both sides of the anode plate to form an anode cell, and the cathode chamber is equipped with a cathode plate and two electrode plate frames arranged at both sides of the cathode plate to form a cathode cell; the TA alkaline salt solution enters the electrolysis assembly from an anode side, and a TA crude slurry is recovered in the anode cell, and an alkaline solution is recovered in the cathode cell, and oxygen and hydrogen are collected separately; each electrode plate frame is in a rectangular shape, wherein one or more arc-shaped corrugated separator is provided along a long side direction of the electrode plate frame, and a curvature diameter of the arc-shaped corrugated separator is not less than a short side size of the cell;
a second solid-liquid separation step, comprising filtering the TA crude slurry to obtain a TA crude product; and
a refining step, comprising recrystallizing the TA crude product to obtain the PTA;
wherein when a first enzymatic hydrolysis cell of the plurality of enzymatic hydrolysis cells completes the enzymatic hydrolysis step and performs the first solid-liquid separation step, a second enzymatic hydrolysis cell of the plurality of enzymatic hydrolysis cells performs the enzymatic hydrolysis step; and after the processing operation on the first enzymatic hydrolysis cell is completed, the second enzymatic hydrolysis cell performs the first solid-liquid separation step, thereby achieving continuous production.

5. The continuous production process for efficient recovery of the PTA from the waste PET of claim 4, further comprising a distillation step, comprising distilling a solution obtained from the first solid-liquid separation step to recover ethylene glycol.

6. The continuous production process for efficient recovery of the PTA from the waste PET of claim 4, wherein the enzymatic hydrolysis step comprises: preparing 10 millimoles per liter, mmol/L, to 1000 mmol/L sodium phosphate buffer, adding 100 grams, g, to 1000g waste PET per liter of the buffer, and adding PET-degrading enzyme crude enzyme solution with a mass of 0.1% to 1% of the buffer, to perform the enzymatic hydrolysis for 5 hours, h, to 25h;
the refining step comprises:
transporting the TA crude product to a slurry tank, and adding a mixed solvent to adjust a mass concentration of the TA crude product to 20 weight percent, wt%, to 35wt%, wherein the mixed solvent is a mixture of water and at least one of dimethylformamide, DMF, diethylformamide, DEF, and dimethyl sulfoxide, DMSO;
heating to 265°C to 280°C and pressurizing to 6.0 megapascals, MPa, to 8.0 MPa to dissolve the TA crude product; and
performing decolorization and removal of metal impurities on an obtained TA crude solution through an adsorption column, subjecting to stepwise crystallization through a 1 to 6 stage cooling-depressurization flash crystallizer, washing and drying to obtain the PTA.

7. The continuous production process for efficient recovery of the PTA from the waste PET of claim 4, wherein a ratio of a long side size to the short side size of the cell is 2:1 to 5:1, and a depth of the cell is 2 millimeters, mm, to 20 mm; a distance between an electrode plate upper edge and an upper edge of the cell is greater than 1/8 of the long side size of the cell, and a distance between an electrode plate lower edge and a lower edge of the cell is not less than 1/4 of the long side size of the cell.
